# EUROPEAN PATENT APPLICATION

(11) **EP 1 269 995 A1**
(43) Date of publication of application: **02.01.2003**
(21) Application number: 01915776.7
(22) Date of filing: 26.03.2001
(51) Int. Cl.: A61K 9/16

(54) **EASY-TO-TAKE GRANULE**

(30) Priority: 27.03.2000 JP 2000086516
(71) Applicant: KYOWA HAKKO KOGYO CO., LTD., Chiyoda-ku, Tokyo 100-8185 (JP)
(72) Inventor: MURAI, Kouji, Pharmaceutical Res. Institute, Sunto-gun, Shizuoka 411-8731 (JP); NARITA, Shoichi, Pharmaceutical Research Institute, Sunto-gun, Shizuoka 411-8731 (JP); OGASA, Takehiro, Kyowa Hakko Kogyo Co. Ltd., Chiyoda-ku, Tokyo 100-8185 (JP)
(74) Representative: Best, Michael, Dr.
(86) International application number: JP0102406
(87) International publication number: WO01072285

(57) **Abstract**

There are provided granules having a good taking property wherein an active ingredient, at least one kind of soluble additive having an average particle size of smaller than 50 µm and at least one kind of disintegrator are contained.

## Description

### Technical Field

The present invention relates to granules which are quickly dissolved or disintegrated in the oral cavity resulting in a form of solution or suspension and also to a method for giving a quickly disintegrating property in the oral cavity to the granules.

### Background Art

In pharmaceuticals and the like, granules have been widely used as a dosage form having a highly broad applicability. Granules are particularly useful in the case of people such as aged people or small children, whose swallowing ability is low, whereby shaped preparations such as tablets are difficult to be taken. Even in the case of adults, granules are easier to take than other dosage forms when the dose becomes high. In addition, granules have an advantage that the dose can be precisely adjusted depending upon the symptom or age and are a dosage form which is suitable for the dispensation.

However, when taking the granules, it is necessary that all granules dispersed in the oral cavity are carried away from the cavity and, therefore, more water is usually required as compared with the case of tablets or capsules. When granules are taken with a small amount of water, they remain in the oral cavity whereupon a bitter taste of the medicament continues or incompatible feeling is continuously given to beneath the tongue or the gingiva. As a result, the person taking them often has an impression that granules are hard to take. Especially, small children may be sometimes resistant to taking the granules.

As mentioned above, although the granules are inherently the dosage form having a high taking property, they are apt to be thought difficult to be taken because much water is necessary upon taking. Such a point is a problem which is much worried about for the pharmaceuticals where necessary dose is to be taken upon necessity and there has been a strong demand for solving the said problem.

Until now, attempts for improving the taking property have been done by adding a sweetener or flavor to the granules but, even though the unpleasant feel upon taking is improved by giving a preference thereto, that is not an essential improvement for the problem of granules that they are hard to take. As a substitute for granules, dry syrup may, for example, be used but dry syrup is bulkier than granules and requires much water in taking it whereby the person who takes it may sometimes have an impression that dry syrup is rather hard to take. In addition, dry syrup is a preparation having a prerequisite that it is used by dissolving or suspending it in a predetermined amount of water and, therefore, when it is taken in the same way as in taking granules, its disintegration and dissolution in digestive tracts do not take place properly whereupon there may be a risk that a sufficient pharmaceutical effect is not achieved or side effects arise.

On the other hand, a quickly disintegrating tablet in the oral cavity which is one of the shaped dosage forms such as tablets is a tablet which is quickly dissolved or disintegrated after taking it whereby it can be easily taken even by aged people and small children (Japanese Published Unexamined Patent Application Nos. 05/271,054 and 08/291,051). However, in such shaped dosage forms, the dose is unable to be adjusted unless they are divided or ground, and they are not appropriate in some cases as a preparation for aged people or small children where the dose is to be adjusted depending upon the symptom or age.

Granular product obtained by a granulating operation during the steps of manufacture of common tablets is sometimes called "granules". Manufacture of such "granules" is carried out with an object of improving the manufacturing efficiency in a tableting operation or a filling operation thereafter or of making the quality better. Those "granules" have no sufficient strength for being distributed on the market and, therefore, they produce powder and make the taking property bad or disturb the dispensation.

Desirable granules are manufactured in such a manner that they have sufficient strength whereby no powder is generated during distribution. Thus, they are different from the "granules" as a granulated product obtained by a granulating operation during the steps of manufacture of tablets, and an art for their manufacture is different as well. Although there is a description of manufacturing a granulated product during the steps for the manufacture of quickly disintegrating tablets in the oral cavity (Japanese Published Unexamined Patent Application No. 11/43,429; WO 97/47287), there is no description of an art for the manufacture of granules as the final form which are quickly disintegrated in the oral cavity.

There has been also known a solid granular preparation making the disintegration in the oral cavity good and having a good strength as a granular preparation (Japanese Published Unexamined Patent Application No. 11/343,231). However, in the said patent, there are only shown the cases where a very limited saccharide selected from erythritol, xylitol and sorbitol is used together with polyvinylpyrrolidone which is a binder. There is another problem therein that the said solid granular preparation is to be contained in a highly air-tight container or bag.

### Disclosure of the Invention

An object of the present invention is to provide granules which are quickly dissolved or disintegrated in the oral cavity becoming a form of solution or suspension so as to improve the taking property of granules and have good strength as a granule, and also to provide a method of giving a quickly disintegrating property in the oral cavity to granules.

In accordance with the present invention, there are provided granules which are quickly dissolved or disintegrated in the oral cavity becoming a form of solution or suspension (hereinafter, they are referred to as "quickly disintegrating granules in the oral cavity"). When the quickly disintegrating granules in the oral cavity become a form of solution or suspension in the oral cavity, the volume is greatly reduced and the scattering thereof in the oral cavity is very little. Therefore, it is possible to take the medicament with a feeling as if saliva were swallowed and, with only a few amount of water being taken, the medicament rarely remains in the oral cavity. Further, since they are in a form of solution or suspension, an incompatible feeling is rarely noted beneath the tongue and at the gingiva.

The present invention relates to the following (1) to (19).
(1) A granule comprising an active ingredient, at least one kind of soluble additive having an average particle size of smaller than 50 µm and at least one kind of disintegrator.
(2) A granule comprising an active ingredient, at least one kind of soluble additive in a form of secondary particles having an average particle size of 30 to 500 µm where powder or crystals having an average particle size of smaller than 50 µm as a primary particle is aggregated and at least one kind of disintegrator.
(3) The granule according to (1) or (2), wherein the said granule is quickly dissolved or disintegrated in the oral cavity.
(4) The granule according to any one of (1) to (3), wherein the soluble additive is at least one member selected from the group consisting of a sugar or a sugar alcohol, an amino acid or a derivative thereof, a polyol, a solubilized cellulose derivative and a soluble epoxy or acrylic polymer.
(5) The granule according to any one of (1) to (3), wherein the soluble additive is a sugar or a sugar alcohol having less than 24 carbon atoms.
(6) The granule according to (5), wherein the sugar or the sugar alcohol is at least one member selected from the group consisting of mannitol, xylitol, sorbitol, erythritol and trehalose.
(7) The granule according to any one of (1) to (6), wherein the disintegrator is at least one member selected from the group consisting of crospovidone, crystalline cellulose-carmellose sodium, sodium carboxymethyl starch and hydroxypropyl starch.
(8) The granule according to any one of (1) to (7), wherein the active ingredient is coated or made into a matrix with one or more coating agents selected from the group consisting of an acrylic polymer, a cellulose polymer, a natural polymer, a fat/oil and a fat/oil salt.
(9) The granule according to any one of (1) to (8), wherein the active ingredient is the one selected from the group consisting of medicaments acting on the autonomic nervous system, medicaments acting on the somatic nervous system, medicaments acting on the smooth muscle, antihistaminic/antiallergic medicaments, medicaments acting on the central nervous system, medicaments acting on the cardiovascular system, diuretic/anti-diuretic medicaments, medicaments acting on the respiratory system, medicaments acting on the digestive system, medicaments acting on the blood/hematopoietic organ, hormones, vitamins, antidotes, anti-infective medicaments, anti-malignant tumor medicaments, immunological medicaments, diagnostic medicaments, and narcotics/stimulants.
(10) The granules according to any one of (1) to (8), wherein the active ingredient is one or more active ingredients selected from the group consisting of acetylspiramycin, amoxicillin, ethyl icosapentate, itraconazole, oxatomide, glybuzole, glutathione, ketophenylbutazone, cobamamide, cisapride, todralazine hydrochloride, tropisetron hydrochloride, domperidone, sodium valproate, fluorouracil, flunarizine hydrochloride, flurazepam hydrochloride, benidipine hydrochloride, minocycline hydrochloride, mebendazole, medroxyprogesterone, ubidecarenone, pyridoxal phosphate and levodopa.
(11) A method for giving a quickly disintegrating property in the oral cavity to granules, characterized in that, an active ingredient is compounded with at least one kind of soluble additive having an average particle size of smaller than 50 µm and at least one kind of disintegrator.
(12) A method for giving a quickly disintegrating property in the oral cavity to granules, characterized in that, an active ingredient is compounded with at least one kind of soluble additive in a form of secondary particles having an average particle size of 30 to 500 µm where powder or crystals having an average particle size of smaller than 50 µm as a primary particle is aggregated and at least one kind of disintegrator.
(13) The method for giving a quickly disintegrating property in the oral cavity to granules according to (11) or (12), wherein the soluble additive is at least one member selected from the group consisting of a sugar or a sugar alcohol, an amino acid or a derivative thereof, a polyol, a solubilized cellulose derivative and a soluble epoxy or acrylic polymer.
(14) The method for giving a quickly disintegrating property in the oral cavity to granules according to (11) or (12), wherein the soluble additive is a sugar or a sugar alcohol having less than 24 carbon atoms.
(15) The method for giving a quickly disintegrating property in the oral cavity to granules according to (14), wherein the sugar or the sugar alcohol is at least one member selected from the group consisting of mannitol, xylitol, sorbitol, erythritol and trehalose.
(16) The method for giving a quickly disintegrating property in the oral cavity to granules according to any one of (11) to (15), wherein the disintegrator is at least one member selected from the group consisting of crospovidone, crystalline cellulose-carmellose sodium, sodium carboxymethyl starch and hydroxypropyl starch.
(17) The method for giving a quickly disintegrating property in the oral cavity to granules according to any one of (11) to (16), wherein the active ingredient is coated or made into a matrix with one or more coating agents selected from the group consisting of an acrylic polymer, a cellulose polymer, a natural polymer, a fat/oil and a fat/oil salt.
(18) The method for giving a quickly disintegrating property in the oral cavity to granules according to any one of (11) to (17), wherein the active ingredient is the one selected from the group consisting of medicaments acting on the autonomic nervous system, medicaments acting on the somatic nervous system, medicaments acting on the smooth muscle, antihistaminic/antiallergic agents, medicaments acting on the central nervous system, medicaments acting on the cardiovascular system, diuretic/anti-diuretic medicaments, medicaments acting on the respiratory system, medicaments acting on the digestive system, medicaments acting on the blood/hematopoietic organ, hormones, vitamins, antidotes, anti-infective medicaments, anti-malignant tumor medicaments, immunological medicaments, diagnostic medicaments and narcotics/stimulants.
(19) The method for giving a quickly disintegrating property in the oral cavity to granules according to any one of (11) to (17), wherein the active ingredient is one or more active ingredients selected from the group consisting of acetylspiramycin, amoxicillin, ethyl icosapentate, itraconazole, oxatomide, glybuzole, glutathione, ketophenylbutazone, cobamamide, cisapride, todralazine hydrochloride, tropisetron hydrochloride, domperidone, sodium valproate, fluorouracil, flunarizine hydrochloride, flurazepam hydrochloride, benidipine hydrochloride, minocycline hydrochloride, mebendazole, medroxyprogesterone, ubidecarenone, pyridoxal phosphate and levodopa.

The granules of the present invention contain at least one kind of soluble additive having an average particle size of smaller than 50 µm and at least one kind of disintegrator. The soluble additive may be in a form of secondary particles having an average particle size of 30 to 500 µm where powder or crystals of an average particle size of smaller than 50 µm as a primary particle is aggregated. The active ingredient may be coated or made into a matrix with a coating agent and, with regard to the coating agent, one or more coating agents selected from the group consisting of an acrylic polymer (the said acrylic polymer has the same meaning as an acrylic polymer in the definition for the coating agent which will be mentioned later), a cellulose polymer, a natural polymer, a fat/oil and a fat/oil salt are used.

In the present invention, a soluble additive means an additive which is soluble in water and a preferred soluble additive has such a characteristic that particles are bonded by physical or electrostatic action etc. Its specific examples are a sugar and a sugar alcohol, an amino acid and a derivative thereof, a polyol such as polyethylene glycol, a solubilized cellulose derivative, a soluble epoxy or acrylic polymer, etc. The soluble acrylic polymer in the exemplification for the soluble additive is a soluble polymer where acrylic acid derivative(s) and/or methacrylic acid derivative(s) are/is polymerized. Among the soluble additives, preferred one is a sugar and a sugar alcohol having less than 24 carbon atoms and more preferred examples are mannitol, xylitol, sorbitol, erythritol and trehalose. When the granules are prepared using the soluble additive, it is now easy to prepare the granules having a characteristic that they are quickly dissolved or disintegrated in the oral cavity and also have a sufficient strength as a granule. The soluble additive is used in an amount of, for example, 50 to 99.9 parts by weight or, preferably, 80 to 95 parts by weight to 100 parts by weight of the granule.

Common methods for the manufacture of powder may be applied as a method for making the soluble additive into a form of powder or crystals where an average particle size is smaller than 50 µm or into a form of secondary particles having an average particle size of 30 to 500 µm where powder or crystals having an average particle size of smaller than 50 µm is aggregated as a primary particle. In some of the soluble additives, common commercially available products as they are may be used in the present invention.

When a soluble additive is ground using various grinding machines such as hammer mill, pin mill, jet grinder or mortar, a soluble additive in a form of powder or crystals having an average particle size of smaller than 50 µm is obtained. On the other hand, when a soluble additive is dissolved in water or in an organic solvent and granulated by a spray drying method, there is obtained a soluble additive in a form of secondary particles having an average particle size of 30 to 500 µm where powder or crystals having an average particle size of smaller than 50 µm as a primary particle is aggregated. It is also possible that, when a soluble additive in a form of powder or crystals having a particle size of smaller than 50 µm is granulated by a common granulating method such as fluidized bed granulation, stirring granulation, tumbling granulation, tumbling fluidized bed granulation or extrusion granulation, a soluble additive in a form of secondary particles having an average particle size of 30 to 500 µm where powder or crystals having an average particle size of smaller than 50 µm as a primary particle is aggregated.

In the present invention, a disintegrator is added with a role of helping the disintegration of granules in the oral cavity, of making the granules previously into an easily-disintegrable structure in the process for the manufacture of granules, etc. The former role is an action of changing the shape by way of swelling, dissolving, etc. that all disintegrator have, in other words, it is the so-called disintegrating action. The latter role is an action that the disintegrator comes to the space among each of the particles of the soluble additive except the disintegrator and the active ingredient whereby, during the process of the manufacture of granules, caking of the particles of the soluble additive except the disintegrator and the active ingredient is prevented and capillary for permeation of saliva into the granule is formed. Examples of the disintegrator are celluloses such as low-substituted hydroxypropyl cellulose and crystalline cellulose; various starches and starch derivatives such as corn starch, starch which is partially made into an α-form, and hydroxypropyl starch; crospovidone; bentonite; etc. More preferred examples are crospovidone, crystalline cellulose-carmellose sodium, sodium carboxymethyl starch and hydroxypropyl starch. The disintegrator is used, for example, in an amount of 0.1 to 20 part(s) by weight, preferably, 1 to 10 part(s) by weight to 100 parts by weight of the granule.

The active ingredient used in the granules of the present invention may be in any form of solid, powder, crystals, oil, solution, etc., and its specific examples are one or more active ingredients selected from the group consisting of medicaments acting on the autonomic nervous system, medicaments acting on the somatic nervous system, medicaments acting on the smooth muscle, antihistaminic/antiallergic medicaments, medicaments acting on the central nervous system, medicaments acting on the cardiovascular system, diuretic/anti-diuretic medicaments, medicaments acting on the respiratory system, medicaments acting on the digestive system, medicaments acting on the blood/hematopoietic organ, hormones, vitamins, antidotes, anti-infective medicaments, anti-malignant tumor medicaments, immunological medicaments, diagnostic agents, narcotics/stimulants, etc.

Pharmacologically acceptable salts of the above-mentioned active ingredient may also be used as the active ingredient, and examples of such a salt are salts with an inorganic acid (e.g. hydrochloric acid, sulfuric acid or nitric acid), an organic acid (e.g. succinic acid, acetic acid, propionic acid or trifluoroacetic acid), an inorganic base (e.g. an alkali metal such as sodium or potassium, or an alkaline earth metal such as calcium or magnesium), an organic base (e.g. an organic amine such as triethylamine or a basic amino acid such as arginine), etc.

More specific examples of the active ingredient of the present invention are one or more active ingredients selected from the group consisting of acetylspiramycin, amoxicillin, ethyl icosapentate, itraconazole, oxatomide, glybuzole, glutathione, ketophenylbutazone, cobamamide, cisapride, todralazine hydrochloride, tropisetron hydrochloride, domperidone, sodium valproate, fluorouracil, flunarizine hydrochloride, flurazepam hydrochloride, benidipine hydrochloride, minocycline hydrochloride, mebendazole, medroxyprogesterone, ubidecarenone, pyridoxal phosphate, levodopa and pharmacologically acceptable salts thereof.

The active ingredient may be coated or made into a matrix with a coating agent depending upon its physicochemical characteristics. Examples of the object for coating or making into a matrix are masking of taste and smell, making the preparation soluble in the intestines, making the active ingredient released gradually, making the granule stabilized by shutting out the light or preventing from moisture, etc., and such coating or making into a matrix may be carried out by known methods. Among them, it is preferred that masking of taste and smell is taken into consideration for improving the taking property. Specific examples of the coating agent used in the present invention are one or more coating agents selected from the group consisting of acrylic polymers(the said acrylic polymer means a polymer where acrylic acid derivative(s) and/or methacrylic acid derivative(s) are/is polymerized) such as methacrylic acid copolymer, aminoalkyl methacrylate copolymer and carboxyvinyl polymer; cellulose polymers such as ethyl cellulose, hydroxypropylmethyl cellulose, hydroxylpropyl cellulose and cellulose acetate phthalate; natural polymers such as gum arabic, pullulan, alginic acid, agar and gelatin; stearic acid; magnesium stearate; hydrogenated oil; paraffin; carnauba wax; fat/oil such as glycerol fatty acid esters; fat/oil salt, etc. Such a coating agent may be used together, if necessary, with a plasticizer or a soluble additive (the said soluble additive has the same meaning as defined above) and, for example, it may be used by mixing it with polyethylene glycol, polyoxyethylenepolyoxypropylene glycol, polyoxyethylene hydrogenated castor oil, triethyl citrate, Polysorbate, a sugar, a sugar alcohol, an oligosaccharide, etc.

The quickly disintegrating granules in the oral cavity of the present invention may contain various additives which are used for the manufacture of common preparations so far as they will not deteriorate the dissolving or disintegrating property or the preparation strength, and the amount thereof may be that which is used for the manufacture of common preparations. Examples of such additives are binders, sour agents, foaming agents, artificial sweeteners, flavors, lubricants, colorants, stabilizers, solubilizers, etc.

Examples of the binder are hydroxypropyl cellulose, hydroxypropylmethyl cellulose, crystalline cellulose, α-starch, polyvinylpyrrolidone, gum arabic powder, gelatin, pullulan, etc. Examples of the sour agent are citric acid, tartaric acid, malic acid, etc. Examples of the foaming agent are sodium bicarbonate etc. Examples of the artificial sweetener are saccharine sodium, dipotassium glycyrrhizinate, aspartame, stevia, thaumatin, etc. Examples of the flavor are lemon, lemon lime, orange, strawberry, vanilla, menthol, etc. Examples of the lubricant are magnesium stearate, sucrose fatty acid esters, polyethylene glycol, talc, stearic acid, etc. Examples of the colorant are food dyes such as Yellow No. 5, Red No. 2 and Blue No. 2, food lake dyes, red iron oxide, etc. Examples of the stabilizer are antioxidants such as tocopherol, sodium sulfite etc.; and when the active ingredient is basic or acidic, its examples are a basic substance and an acidic substance, respectively. Examples of the solubilizer are cyclodextrin, Polysorbate, sodium laurylsulfate, etc.

In the manufacture of the granules of the present invention, a method which is commonly used for the manufacture of granules such as fluidized bed granulation, stirring granulation, tumbling granulation, tumbling fluidized bed granulation, extrusion granulation, etc., is carried out using an active ingredient (the said active ingredient may be coated or made into a matrix with a coating agent), a soluble additive which is in a form of powder or crystals having an average particle size of smaller than 50 µm or in a form of secondary particles having an average particle size of 30 to 500 µm where powder or crystals having an average particle size of smaller than 50 µm as a primary particle is aggregated and a disintegrator.

It is preferred to previously mix well the active ingredient, the soluble additive and the disintegrator to which other components such as a binder may be added if necessary. Mixing may be carried out by a commonly used mixing method and, when mixing of the soluble additive with the disintegrator is insufficient, there may be formed a strong caking of particles of the soluble additive. After mixing, granulation is carried out using purified water, ethanol, etc. When the granulation is carried out by means of fluidized bed granulation, stirring granulation, tumbling granulation, tumbling fluidized bed granulation, extrusion granulation, etc., a necessary amount of purified water, ethanol, etc., is added for granulation into a desired size. At that time, purified water, ethanol, etc., may be added together with the binder. After the granulation, drying is carried out by a commonly used method. After the granulation or drying, there is no need for conducting a treatment such as preservation under a special circumstance such as a wet circumstance. When the dried granulated products are classified by means of a desirable sieve or the like, the granules are prepared. When the sieve mesh is adjusted at that time, it is possible to prepare each of pills, granules, fine particles and diluted powder as mentioned in the Japanese Pharmacopoeia. Antistatic agents, corrigents for taste and smell, etc., may be added to the resulting granules. Alternatively, the granules may be filled in a bottle or packed as a separately packed product.

The granules prepared as such are granules (quickly disintegrating granules in the oral cavity) having a sufficient strength and a good taking property.

The present invention further relates to a method of giving a quickly disintegrating property in the oral cavity to granules, characterized in that, an active ingredient is compounded with at least one kind of soluble additive having an average particle size of smaller than 50 µm and at least one kind of disintegrator. With regard to a method of giving a quickly disintegrating property in the oral cavity to granules in accordance with the present invention, the above-mentioned method for the manufacture of quickly disintegrating granules in the oral cavity may be applied as it is.

The present invention will now be more specifically illustrated by way of the following Examples and Comparative Examples.

### Best Modes for Carrying Out the Invention

### <Example 1>

D-Mannitol (Towakasei Co., Ltd.: Mannit P: average particle size of about 60 µm) was previously ground by a hammer mill (manufactured by Fuji Paudal Co., Ltd.: Sample Mill) to give D-mannitol having an average particle size of about 30 µm. The ground D-mannitol (90 g), 5.5 g of crospovidone (GAF: Polyplasdon XL-10), 2 g of hydroxypropyl cellulose and 2 g of oxatomide (a therapeutic agent for allergic diseases) were mixed in a mortar and kneaded after addition of purified water in an amount as shown in the table. The kneaded product was granulated using an extrusion granulator (manufactured by Fuji Paudal Co., Ltd.: Domegran) and dried in a drier (manufactured by Ikeda Rika: Drying Oven) to give granules.

### <Example 2>

D-Mannitol (Towakasei Co., Ltd.: Mannit P: average particle size of about 60 µm) was previously ground by a high-speed jet grounding machine (manufactured by Nippon Pnewmatic Mfg. Co., Ltd.: Jet Mill PJM-1-3) to give D-mannitol having an average particle size of about 20 µm. The ground D-mannitol (90 g), 5.5g of crospovidone (GAF: Polyplasdon XL-10), 2 g of hydroxypropyl cellulose and 2 g of oxatomide (a therapeutic agent for allergic diseases) were mixed in a mortar and kneaded after addition of purified water in an amount as shown in the table. The kneaded product was granulated using an extrusion granulator (manufactured by Fuji Paudal Co., Ltd.: Domegran) and dried in a drier (manufactured by Ikeda Rika: Drying Oven) to give granules.

### <Example 3>

D-Mannitol (Towakasei Co., Ltd.: Mannit P: average particle size of about 60 µm) was dissolved in water and the solution was spray-dried using a spray drier (manufactured by Ohkawara Kakohki Co., Ltd.: Spray Drier ML-12-BS-12) to give D-mannitol having an average particle size of about 30 µm. The ground D-mannitol (90 g), 5.5 g of crospovidone (GAF: Polyplasdon XL-10), 2 g of hydroxypropyl cellulose and 2 g of oxatomide (a therapeutic agent for allergic diseases) were mixed in a mortar and kneaded after addition of purified water in an amount as shown in the table. The kneaded product was granulated using an extrusion granulator (manufactured by Fuji Paudal Co., Ltd. : Domegran) and dried in a drier (manufactured by Ikeda Rika: Drying Oven) to give granules.

### <Example 4>

Commercially available spray-dried D-mannitol (Roquett: PEARLITOL: in a form of second particles with an average particle size of 200 µm where particles having an average particle size of smaller than 50 µm were aggregated) (90 g), 5.5 g of crospovidone (GAF: Polyplasdon XL-10), 2 g of hydroxypropyl cellulose and 2 g of oxatomide (a therapeutic agent for allergic diseases) were mixed in a mortar and kneaded after addition of purified water in an amount as shown in the table. The kneaded product was granulated using an extrusion granulator (manufactured by Fuji Paudal Co., Ltd.: Domegran) and dried in a drier (manufactured by Ikeda Rika: Drying Oven) to give granules.

### <Comparative Example 1>

D-Mannitol (Towakasei Co Ltd. : Mannit P: average particle size of about 60 µm) as it was (90 g), 5.5 g of crospovidone (GAF: Polyplasdon XL-10), 2 g of hydroxypropyl cellulose and 2 g of oxatomide (a therapeutic agent for allergic diseases) were mixed in a mortar and kneaded after addition of purified water in an amount as shown in the table. The kneaded product was granulated using an extrusion granulator (manufactured by Fuji Paudal Co., Ltd.: Domegran) and dried in a drier (manufactured by Ikeda Rika: Drying Oven) to give granules.

### <Example 5>

D-Mannitol having an average particle size of about 30 µm, which was the same as in Example 3 (91.2 g), 5.5 g of crospovidone (GAF: Polyplasdone XL-10), 2 g of hydroxypropyl cellulose and 0.8 g of benidipine hydrochloride (a therapeutic agent for angina pectoris) were mixed in a mortar and kneaded after addition of 24 ml of purified water. The kneaded product was granulated using an extrusion granulator (manufactured by Fuji Paudal Co., Ltd.: Domegran) and dried in a drier (manufactured by Ikeda Rika: Drying Oven) to give granules.

### <Example 6>

D-Mannitol having an average particle size of about 30 µm, which was the same as in Example 3 (89.95 g), 5.5 g of crospovidone (GAF: Polyplasdone XL-10), 2 g of hydroxypropyl cellulose, 0.05 g of aspartame and 2 g of domperidone (an agent for improving the movement of digestive tracts) were mixed in a mortar and kneaded after addition of 24 ml of purified water. The kneaded product was granulated using an extrusion granulator (manufactured by Fuji Paudal Co., Ltd.: Domegran) and dried in a drier (manufactured by Ikeda Rika: Drying Oven) to give granules.

### <Comparative Example 2>

D-Mannitol having an average particle size of about 30 µm, which was the same as in Example 3 (95.5 g), 2 g of hydroxypropyl cellulose and 2 g of oxatomide (a therapeutic agent for allergic diseases) were mixed in a mortar and kneaded after addition of 24 ml of purified water. The kneaded product was granulated using an extrusion granulator (manufactured by Fuji Paudal Co., Ltd.: Domegran) and dried in a drier (manufactured by Ikeda Rika: Drying Oven) to give granules.

Results of evaluation of strength and disintegrating property of the granules of the present invention are shown in Table 1 and Table 2.

In the tables, "wetting liquid amount" shows the amount of water which was added in the kneading of 99.5 g of powder while strength of the granules was evaluated from the generating rate (%) of fine particles when 1 g of the granules was placed in a test tube of 15 mm inner diameter and shaken up and down for 60 minutes with a shaking width of 40 mm at a rate of 300 times per minute (empirically, the generating rate of fine particles is preferred to be less than 5%). Disintegrating property was evaluated from an average disintegrating time (in seconds) when the disintegrating test according to the Japanese Pharmacopoeia XIII was carried out (empirically, the average disintegrating time is preferred to be within 15 seconds).

**Table 1**

| | Example 1 | | Example 2 | | Example 3 | | Example 4 | | Comp. Ex. 1 | |
|---|---|---|---|---|---|---|---|---|---|---|
| WLA | strength | DP | strength | DP | strength | DP | strength | DP | strength | DP |
| 12 ml | 18.7% | 7 sec | 9.6% | 5 sec | - | - | - | - | 15.1% | 8 sec |
| 15 ml | 8.3% | 11 sec | 3.2% | 7 sec | 10.6% | 4 sec | 11.8% | 15 sec | 8.2% | 13 sec |
| 18 ml | 3.8% | 14 sec | 2.3% | 10 sec | 5.2% | 11 sec | 4.3% | 19 sec | 3.7% | 17 sec |
| 21 ml | 3.2% | 18 sec | 2.0% | 14 sec | 3.8% | 7 sec | 3.1% | 20 sec | 2.3% | 23 sec |
| 24 ml | 2.0% | 21 sec | 1.5% | 18 sec | 2.4% | 9 sec | 1.6% | 19 sec | 1.8% | 29 sec |
| 27 ml | 1.6% | 24 sec | 1.4% | 30 sec | 2.4% | 12 sec | 0.9% | 15 sec | - | - |
| 30 ml | - | - | - | - | 2.0% | 13 sec | 0.4% | 15 sec | - | - |
| WLA: Wetting liquid amount | | | | | | | | | | |
| DP: Disintegrating property | | | | | | | | | | |

It was possible to prepare the granules having a sufficient strength (generating rate of the fine particles was less than 5%) and a good disintegrating property (disintegrating time was shorter than 15 seconds) in Examples 1 to 4 where at least one kind of soluble additive and disintegrator were mixed and, with regard to the soluble additive, there was used either that in the form of powder or crystals having an average particle size of smaller than 50 µm or that in the form of secondary particles having an average particle size of 30 to 500 µm where powder or crystals having an average particle size of smaller than 50 µm as a primary particle was aggregated. However, in Comparative Example 1 where an average particle size was larger than 50 µm, it was not possible to prepare the granules having a sufficient strength and a good disintegrating rate.

**Table 2**

| | Example 3 | | Example 5 | | Examples 6 | | Comp. Ex. 2 | |
|---|---|---|---|---|---|---|---|---|
| WLA | strength | DP | strength | DP | strength | DP | strength | DP |
| 24 ml | 2.4% | 9 sec | 3.6% | 10 sec | 2.5% | 11 sec | 2.1% | 42 sec |
| WLA: Wetting liquid amount | | | | | | | | |
| DP: Disintegrating property | | | | | | | | |

It was also possible to prepare the granules having a sufficient strength and a good disintegrating property even in Examples 5 and 6 where the medicament was changed from Example 3. However, in Comparative Example 2 where no disintegrator was contained, it was not possible to prepare the granules having a sufficient strength and a good disintegrating property.

### Industrial Applicability

In accordance with the present invention, it is possible to provide granules (quickly disintegrating granules in the oral cavity) which are quickly dissolved or disintegrated in the oral cavity giving a form of solution or suspension. The said quickly disintegrating granules in the oral cavity are excellent as a means for improving the taking property of granules and, in addition, they have strength necessary for granules.

## Claims

1. A granule comprising an active ingredient, at least one kind of soluble additive having an average particle size of smaller than 50 µm and at least one kind of disintegrator.

2. A granule comprising an active ingredient, at least one kind of soluble additive in a form of secondary particles having an average particle size of 30 to 500 µm where powder or crystals having an average particle size of smaller than 50 µm as a primary particle is aggregated and at least one kind of disintegrator.

3. The granule according to claim 1 or 2, wherein the said granule is quickly dissolved or disintegrated in the oral cavity.

4. The granule according to any one of claims 1 to 3, wherein the soluble additive is at least one member selected from the group consisting of a sugar or a sugar alcohol, an amino acid or a derivative thereof, a polyol, a solubilized cellulose derivative and a soluble epoxy or acrylic polymer.

5. The granule according to any one of claims 1 to 3, wherein the soluble additive is a sugar or a sugar alcohol having less than 24 carbon atoms.

6. The granule according to claim 5, wherein the sugar or the sugar alcohol is at least one member selected from the group consisting of mannitol, xylitol, sorbitol, erythritol and trehalose.

7. The granule according to any one of claims 1 to 6, wherein the disintegrator is at least one member selected from the group consisting of crospovidone, crystalline cellulose-carmellose sodium, sodium carboxymethyl starch sodium and hydroxypropyl starch.

8. The granule according to any one of claims 1 to 7, wherein the active ingredient is coated or made into a matrix with one or more coating agents selected from the group consisting of an acrylic polymer, a cellulose polymer, a natural polymer, a fat/oil and a fat/oil salt.

9. The granule according to any one of claims 1 to 8, wherein the active ingredient is the one selected from the group consisting of medicaments acting on the autonomic nervous system, medicaments acting on the somatic nervous system, medicaments acting on the smooth muscle, antihistaminic/antiallergic medicaments, medicaments acting on the central nervous system, medicaments acting on the cardiovascular system, diuretic/anti-diuretic medicaments, medicaments acting on the respiratory system, medicaments acting on the digestive system, medicaments acting on the blood/hematopoietic organ, hormones, vitamins, antidotes, anti-infective medicaments, anti-malignant tumor medicaments, immunological medicaments, diagnostic medicaments and narcotics/stimulants.

10. The granule according to any one of claims 1 to 8, wherein the active ingredient is one or more active ingredients selected from the group consisting of acetylspiramycin, amoxicillin, ethyl icosapentate, itraconazole, oxatomide, glybuzole, glutathione, ketophenylbutazone, cobamamide, cisapride, todralazine hydrochloride, tropisetron hydrochloride, domperidone, sodium valproate, fluorouracil, flunarizine hydrochloride, flurazepam hydrochloride, benidipine hydrochloride, minocycline hydrochloride, mebendazole, medroxyprogesterone, ubidecarenone, pyridoxal phosphate and levodopa.

11. A method for giving a quickly disintegrating property in the oral cavity to granules, **characterized in that**, an active ingredient is compounded with at least one kind of soluble additive having an average particle size of smaller than 50 µm and at least one kind of disintegrator.

12. A method for giving a quickly disintegrating property in the oral cavity to granules, **characterized in that**, an active ingredient is compounded with at least one kind of soluble additive in a form of secondary particles having an average particle size of 30 to 500 µm where powder or crystals having an average particle size of smaller than 50 µm as a primary particle is aggregated and at least one kind of disintegrator.

13. The method for giving a quickly disintegrating property in the oral cavity to granules according to claim 11 or 12, wherein the soluble additive is at least one member selected from the group consisting of a sugar or a sugar alcohol, an amino acid or a derivative thereof, a polyol, a solubilized cellulose derivative and a soluble epoxy or acrylic polymer.

14. The method for giving a quickly disintegrating property in the oral cavity to granules according to claim 11 or 12, wherein the soluble additive is a sugar or a sugar alcohol having less than 24 carbon atoms.

15. The method for giving a quickly disintegrating property in the oral cavity to granules according to claim 14, wherein the sugar or the sugar alcohol is at least one member selected from the group consisting of mannitol, xylitol, sorbitol, erythritol and trehalose.

16. The method for giving a quickly disintegrating property in the oral cavity to granules according to any one of claims 11 to 15, wherein the disintegrator is at least one member selected from the group consisting of crospovidone, crystalline cellulose-carmellose sodium, sodium carboxymethyl starch and hydroxypropyl starch.

17. The method for giving a quickly disintegrating property in the oral cavity to granules according to any one of claims 11 to 16, wherein the active ingredient is coated or made into a matrix with one or more coating agents selected from the group consisting of an acrylic polymer, a cellulose polymer, a natural polymer, a fat/oil and a fat/oil salt.

18. The method for giving a quickly disintegrating property in the oral cavity to granules according to any one of claims 11 to 17, wherein the active ingredient is the one selected from the group consisting of medicaments acting on the autonomic nervous system, medicaments acting on the somatic nervous system, medicaments acting on the smooth muscle, antihistaminic/antiallergic medicaments, medicaments acting on the central nervous system, medicaments acting on the cardiovascular system, diuretic/anti-diuretic medicaments, medicaments acting on the respiratory system, medicaments acting on the digestive system, medicaments acting on the blood/hematopoietic organ, hormones, vitamins, antidotes, anti-infective medicaments, anti-malignant tumor medicaments, immunological medicaments, diagnostic medicaments and narcotics/stimulants.

19. The method for giving a quickly disintegrating property in the oral cavity to granules according to any one of claims 11 to 17, wherein the active ingredient is one or more active ingredients selected from the group consisting of acetylspiramycin, amoxicillin, ethyl icosapentate, itraconazole, oxatomide, glybuzole, glutathione, ketophenylbutazone, cobamamide, cisapride, todralazine hydrochloride, tropisetron hydrochloride, domperidone, sodium valproate, fluorouracil, flunarizine hydrochloride, flurazepam hydrochloride, benidipine hydrochloride, minocycline hydrochloride, mebendazole, medroxyprogesterone, ubidecarenone, pyridoxal phosphate and levodopa.
